# EUROPEAN PATENT SPECIFICATION

(11) **EP 0 405 658 B1**
(45) Date of publication and mention of the grant of the patent: **30.08.1995**
(21) Application number: 90201623.7
(22) Date of filing: 20.06.1990
(51) Int. Cl.: B29C 65/06, A61M 25/00

(54) **Method and device for mutual connection of tubes**
Verfahren und Vorrichtung zur gegenseitigen Verbindung von Rohren
Procédé et dispositif pour le raccordement mutuel de tuyaux

(30) Priority: 29.06.1989 NL 8901654
(43) Date of publication of application: 02.01.1991
(73) Proprietor: CORDIS EUROPA N.V., NL-9301 LJ Roden (NL)
(72) Inventor: Jansman, Gerardus Antonius Theodorus, NL-9301 VW Roden (NL); Wegereef, Hendrikus Willem, NL-9321 CD Peize (NL)
(74) Representative: 't Jong, Bastiaan Jacobus

(56) References cited:
- EP-A- 0 100 506
- EP-A- 0 145 139
- DE-A- 849 481
- GB-A- 839 507
- GB-A- 1 053 148
- GB-A- 1 239 026
- GB-A- 2 046 866
- GB-A- 2 207 882
- US-A- 3 452 914
- US-A- 3 504 425

## Description

The invention relates to a method for the mutual connection in lengthwise direction of two tube-like elements of thermoplastic material, as defined in the introductory part of claim 1.

Such a method is known from US-A-3 452 914.

The invention has for its object to improve this known method for use in the manufacturing of angiographic catheters, and especially for connecting a basic body and an end part of an angiographic catheter to one another.

Angiographic catheters have very small diameters and especially the end part thereof is very flexible, so that manipulation of the catheter parts for friction-welding is very difficult.

With the inventive method as characterized in the characterizing portion of claim 1, angiographic catheters can be friction-welded in a reliable way. The basic body can easily be handled because it still contains the core wire resulting from the manufacture thereof. The core wire by its very nature has exactly the right dimensions for use as core in the friction-welding process. The through-going channel or lumen in the catheter at the place of the connection of the basic body and the end part will be continuous without any cross-sectional changes.

The invention also relates to and provides a device for rotational butt-welding of a basic body and an end part of thermoplastic material of an angiographic catheter. This device is characterized in claim 4.

The invention will be further elucidated in the following description with reference to the annexed figures.

Fig. 1-3 are a schematic elucidation of the principles of the butt-welding method.

Fig. 4 is a partly broken away and schematic perspective view of a device as according to a preferred embodiment of the invention.

Fig. 5-8 clarify the different stages in the operation of the device of fig. 4.

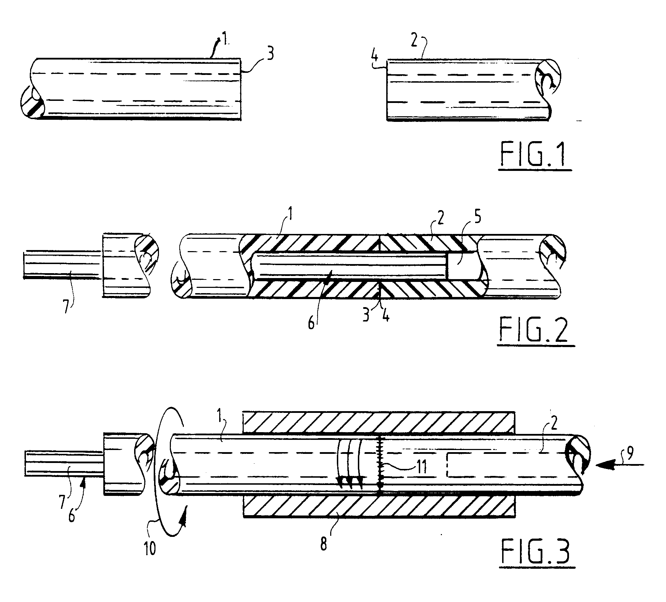

The method is intended for mutual connection in lengthwise direction of a first tube 1 and a second tube 2, the ends of which are shown in fig. 1. The tubes 1 and 2 are of a thermoplastic material.

For application of the method axial end faces 3, 4 are formed on each tube 1, 2. These axial end faces 3, 4 are usually already formed during cutting of the tubes 1, 2 from a greater length of base material therefor.

As shown in fig. 2, in a subsequent step of the method, the tubes 1, 2 are positioned abutting one another with their end faces 3, 4. Inserted into the communicating central channels 5 of the tube portions 1, 2 is a close-fitting core 6. This core 6 is for instance a metal wire or rod with an outer diameter corresponding to the inner diameter of the central channel 5. As fig. 2 shows, the core 6 is arranged with this embodiment of the method such that it extends entirely through the tube 1 and over a determined distance into the tube 2. An end 7 of the core 6 here protrudes outside the tube 1 so that the core 6 can be taken hold of afterwards to remove it from the mutually connected tubes. At the position of the abutting end faces 3, 4 a sleeve 8 is moreover arranged around the tubes 1, 2 as indicated in fig. 3. The sleeve 8 has an internal channel with a diameter corresponding to the outer diameter of the tubes 1, 2 to be connected.

With the tubes 1, 2 enclosed in radial direction between the core 6 and the sleeve 8 at the position of the two abutting end faces 3, 4, the tube 1 is rotated relative to tube 2, as designated with the arrow 10, while the two tubes 1, 2 are urged in axial direction towards one another, as designated with the arrow 9. Because of the relative rotation the end faces 3, 4 slide against one another. Because the faces 3, 4 are here pressed against one another, friction occurs. This friction causes generation of heat whereby the temperature of the material of the tubes 1, 2 increases at the location of the end faces 3, 4. The rotation and axial pressing is maintained until the temperature exceeds the softening temperature of the material of which the tubes 1, 2 consist. In this situation the material of the tubes 1, 2 fuses, whereby a weld 11 is formed. By now stopping the rotation and allowing the weld 11 to cool a strong joint is formed between the tubes 1, 2. During cooling the axial force 9 is preferably maintained.

Due to the enclosure between the core 6 and the sleeve 8 no material can flow away in radial direction. The tubes 1, 2 welded together in this way therefore display no bulges or interruptions at the position of the weld 11.

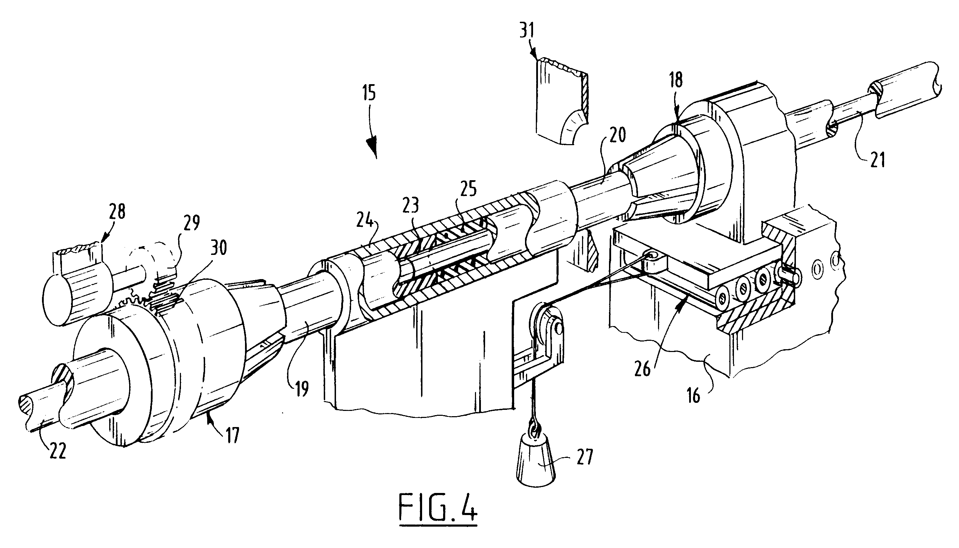

The device 15 according to the invention shown schematically in fig. 4 is intended for use in the manufacture of angiographic catheters. This device 15 comprises a frame 16 whereto are connected first holding means in the form of a chuck 17 for holding fixedly a catheter end part 19 and second holding means in the form of a chuck 18 for fixedly holding a catheter basic body 20. The chucks 17, 18 are self-centering so that they can hold the catheter parts 19, 20 in place directed along an axis. Arranged between the chucks 17 and 18 is a sleeve 24 which is fixedly connected to the frame 16 and coaxial to the said axis.

The catheter body 20 is formed on a metal core wire 21. For connecting the end part 19 to the body 20 the core wire 21 is not removed as yet but is used as core for connection thereto of a catheter end part using the method according to the invention.

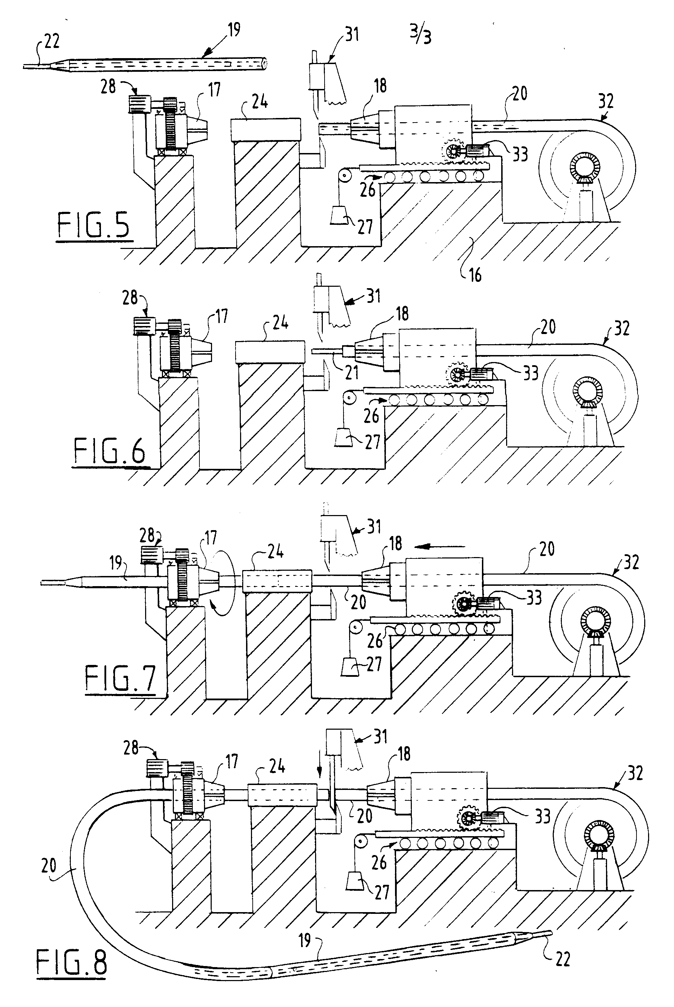

The various phases in the operation of the device 15 are depicted schematically in the figures 5-8. The material for the basic body 20 of the catheters for manufacture is produced in a great length using a per se known method and device and wound onto a reel 32. As noted, this material is formed on a core wire 21. The base material wound onto the reel 32 still contains this metal core wire.

The chuck 18 is mounted on frame 16 for movement in the direction of the axis by means of a roller guiding 26. The chuck 18 can be constrained with a constant force to the left as seen in the figure. In the drawings this constant force is generated using a weight 27.

In order to be able to move the chuck 18 to the right counter to the force exerted by the weight 27 a retracting device 33 is arranged as shown schematically. This enables by using a pinion onto a rack connected fixedly to the head 18.

The catheter basic body 20 that is still connected to the stock on the reel 32 is taken up in the chuck 18 and an axial end face is arranged by means of a cutting device 31. The catheter end part 19 to be connected to the basic body 20 is for example likewise cut off from a greater length and a support wire 22 is inserted into a central channel thereof. The support wire 22 is arranged in the central end face up to a distance from the end face for connection.

As shown in fig. 6, an end portion is removed from the basic body 20 clamped in the chuck 18 so that the core wire 21 protrudes over a distance.

As shown in fig. 7, the body is now inserted into the sleeve 24 and the catheter end part 19 is pushed with its central channel 23 onto the protruding portion of the core wire 21 and likewise received in the sleeve. The catheter end part 19 is here clamped fixedly in the chuck 17. The basic body 20 is in this situation likewise clamped fixedly in the chuck 18. To form the weld the chuck 17 is now set into rotation using the rotating device 28. The rotating device 28 comprises as shown in fig. 4 a motor-driven pinion 29 that is in engagement with a toothing on the periphery of the chuck 17. At the same time the weight 27 is active, whereby the basic body 20 is constrained in the direction to the left. At the position of the contact surface between catheter end part 19 and catheter basic body 20 a rotation friction heat is developed as a result of the friction, whereby the weld connection between the basic body 20 and the end part 19 as described with reference to fig. 1-3 is effected.

After an experimentally determined period the rotating means 28 are switched off while the chuck 18 still remains urged to the left under the influence of the weight 27. The softened or melted material of the end part and body which has flown together cools off whereby this melted material once again hardens. Due to the continued pressing force the occurrence of cavities, for instance as a result of shrinkage, is prevented. It is thus of importance herein that the core wire 21 and the support wire 22 do not abut one another in order not to obstruct the feed in the direction of the pressing force.

After the weld has cooled sufficiently the chucks 17 and 18 are released and a length of base material is pushed through such as is required for the catheter to manufacture. The base material is subsequently cut off using the cutting device 31. The assembly of catheter basic body and catheter end part 19 is further finished hereafter, which includes among other things the removal of core and support wires 21, 22. A subsequent weld operation can then be performed beginning with the step as designated in fig. 5.

As fig. 4 also shows the basic body 20 is of a type comprising a reinforcing layer 25 of woven metal wire. As remarked earlier, no material flow occurs in the direction transversely of the axis during the welding operation so that close to the weld the reinforcing layer 25 maintains its radial position and no wires of the reinforcing layer will protrude inwards or outwards after the welding operation.

The support wire 22 has the purpose of enabling the catheter end part 19 to be clamped stably in the chuck 17, without the central channel 23 thereby being pressed shut. The support wire 22 may have a slightly smaller diameter than the central channel 23 in order to be able to push it in easily. The core wire 21 only needs to be inserted into the catheter end part 19 over a short distance so that a smooth transition results between the central channel of the end part 19 to the central channel of the basic body 20. The invention can further be modified depending on the tube parts for to be connected . One or both tube parts can thus be heated beforehand at the position of the contact surfaces or be additionally heated during welding. In the case of types of material that are difficult to connect to each other directly, an intermediate tube portion can be used which connects readily to each of the materials. This intermediate tube portion can be very short and be arranged in one operation between two tube parts to be connected.

## Claims

1. Method for the mutual connection in lengthwise direction of a first tube (20) and a second tube (19) of thermoplastic material, comprising the forming of an axial end face on each tube (19, 20), positioning the tubes with the axial end faces against one another wherein in communicating central channels thereof a close-fitting core (21) is arranged and a close-fitting sleeve (24) is arranged around the tubes, rotating at least one of the tubes relative to the other and at the same time urging the tubes in axial direction towards one another in order to heat the end faces through frictional heat to a softening temperature of the plastic, wherein close to the end faces the tubes are held enclosed in radial direction between the core (21) and the sleeve (24), ending the rotation, allowing the tubes (19, 20) to cool at least slightly, and removing the core (21) and the sleeve (24), whereby the tubes are butt-welded to one another, **characterized in that** the first tube is a body (20) and the second tube an end part (19) of an angiographic catheter and in that the basic body (20) comprises in the central channel a metal core wire (21) resulting from the manufacture thereof, wherein a portion of the body (20) is removed from the core wire (21) and onto the resulting protruding portion of the core wire (21) the catheter end part (19) is pushed so that the core wire (21) forms the core during rotational butt-welding of the end part (19) to the basic body (20).

2. Method as claimed in claim 1, wherein the basic body (20) is of the type comprising an embedded reinforcing layer of thin, braided wire.

3. Method as claimed in claim 1, wherein the tubes are urged towards one another with a constant force.

4. Device (15) for rotational butt-welding of a basic body (20) and an end part (19) of thermoplastic material of an angiographic catheter comprising a frame (16), first holding means (18) connected to the frame (16) for fixedly holding an end of the basic body (20) directed along an axis, second holding means (17) connected to the frame (16) for fixedly holding the catheter end part (19) directed along the axis, a sleeve (24) disposed coaxially to the axis between the first (18) and second (17) holding means, wherein the second holding means (17) is connected to the frame (16) for rotation along the axis and capable of being rotated by rotation drive means (28), said first holding means (18) being connected to the frame (16) for movement towards the other and capable of being urged towards this other by loading means (27).

## Patentansprüche

1. Verfahren zur gegenseitigen Längsverbindung eines ersten Rohres (20) mit einem zweiten Rohr (19) aus thermoplastischem Material, mit den folgenden Schritten: jedes Rohr (19, 20) wird mit einer axialen Stirnfläche versehen, es werden die Rohre mit ihren axialen Stirnflächen gegeneinander gefügt, wobei in den miteinander in Verbindung stehenden Mittelkanälen der Rohre ein Kern (21) im Paßsitz angeordnet ist und eine dichtpassende Hülse (24) um die Rohre gefügt ist, es wird wenigstens eines der Rohre relativ zu dem anderen gedreht, und gleichzeitig werden die Rohre in Axialrichtung gegeneinandergedrückt, um die Stirnflächen durch Reibungswärme auf eine Erweichungstemperatur des Plastikmaterials zu erhitzen, wobei dicht an den Stirnflächen die Rohre in Radialrichtung zwischen Kern (21) und Hülse (24) umschlossen gehalten werden, es wird die Drehung beendet, und man läßt die Rohre (19, 20) wenigstens etwas abkühlen und entfernt den Kern (21) und die Hülse (24), wodurch die Rohre stumpf miteinander verschweißt werden,
dadurch gekennzeichnet, daß das erste Rohr ein Grundkörper (20) und das zweite Rohr ein Endteil (19) eines angiographischen Katheters ist und daß der Grundkörper (20) in seinem Mittelkanal einen metallischen Kerndraht (21) aufweist, der von der Herstellung herrührt, wobei ein Abschnitt des Körpers (20) vom Kerndraht (21) abgenommen wird und auf den sich ergebenden vorstehenden Abschnitt des Kerndrahtes (21) der Katheterendteil (19) aufgeschoben wird, so daß der Kerndraht (21) während der rotierenden Stumpfverschweißung von Endteil (19) am Grundkörper (20) den Kern bildet.

2. Verfahren nach Anspruch 1, bei welchem im Grundkörper (20) eine Verstärkungsschicht aus dünnem Drahtgeflecht eingebettet ist.

3. Verfahren nach Anspruch 1, bei welchem die Rohre mit einer konstanten Kraft gegeneinandergedrückt werden.

4. Vorrichtung (15) zum Rotations-Stumpfverschweißen eines Grundkörpers (20) mit einem Endteil (19) aus thermoplastischem Material für ein angiographisches Katheter, welche folgende Teile aufweist: einen Rahmen (16), eine erste Haltevorrichtung (18), die mit dem Rahmen (16) verbunden ist, um ein Ende des Grundkörpers (20) zu haltern, der längs einer Achse ausgerichtet ist, eine zweite Haltevorrichtung (17), die mit dem Rahmen (16) verbunden ist, um den Katheterendteil (19) längs der Achse zu haltern, eine Hülse (24), die koaxial zur Achse zwischen der ersten (18) und der zweiten (17) Haltevorrichtung angeordnet ist, wobei die zweite Haltevorrichtung (17) mit dem Rahmen (16) drehbar um die Achse verbunden und durch einen Antrieb (28) drehbar ist, und wobei die erste Haltevorrichtung (18) mit dem Rahmen (16) so verbunden ist, daß eine gegenseitige Bewegung aufeinander zu stattfindet, wobei die beiden Teile durch eine Belastungseinrichtung (27) gegeneinandergedrückt werden.

## Revendications

1. Procédé pour le raccordement mutuel dans le sens de la longueur d'un premier tube (20) et d'un second tube (19) en matière thermoplastique, comprenant la formation d'une face d'extrémité axiale sur chaque tube (19, 20), le positionnement des tubes, les faces d'extrémité axiale étant l'une contre l'autre, et dans lesquelles un noyau ajusté serré (21) est disposé dans les lumières centrales de communication de celles-ci, et un manchon ajusté serré (24) est disposé autour des tubes, la mise en rotation d'au moins l'un des tubes par rapport à l'autre et en même temps la poussée des tubes dans le sens axial l'un vers l'autre afin de chauffer les faces d'extrémité par la chaleur de frottement jusqu'à une température de ramollissement de la matière plastique, dans lequel les tubes sont maintenus enfermés dans le sens radial entre le noyau (21) et le manchon (24) à proximité des faces d'extrémité, l'arrêt de la rotation, la possibilité que les tubes (19, 20) se refroidissent au moins légèrement, et le retrait du noyau (21) et du manchon (24), grâce à quoi les tubes sont soudés en bout l'un à l'autre, caractérisé en ce que le premier tube est un corps (20) et le second tube est une partie d'extrémité (19) d'un cathéter angiographique et en ce que le corps de base (20) comprend un fil noyau métallique (21) dans sa lumière centrale qui résulte de la fabrication de celui-ci, dans lequel une partie du corps (20) est enlevée du fil noyau (21) et la partie d'extrémité (19) de cathéter est poussée sur la partie résultante en saillie du fil noyau (21) de telle façon que le fil noyau (21) forme le noyau durant le soudage en bout rotationnel de la partie d'extrémité (19) sur le corps de base (20).

2. Procédé selon la revendication 1, dans lequel le corps de base (20) est du type comprenant une couche de renforcement encastrée en fil mince tressé.

3. Procédé selon la revendication 1, dans lequel les tubes sont poussés l'un vers l'autre par une force constante.

4. Dispositif (15) pour le soudage en bout rotationnel du corps de base (20) et de la partie d'extrémité (19) en matière thermoplastique d'un cathéter angiographique comprenant un cadre (16), un premier moyen de support (18) relié au cadre (16) pour maintenir fermement une extrémité du corps de base (20) dans le sens d'un axe, un second moyen de support (17) relié au cadre (16) pour maintenir fermement la partie d'extrémité de cathéter (19) dans le sens de l'axe, un manchon (24) disposé coaxialement par rapport à l'axe entre les premier (18) et second (17) moyens de support, dans lequel le second moyen de support (17) est relié au cadre (16) pour pouvoir tourner le long de l'axe et pour pouvoir être mis en rotation par des moyens d'entraînement en rotation (28), ledit premier moyen de support (18) étant relié au cadre (16) pour se déplacer vers l'autre et pouvoir être poussé vers cet autre par un moyen de chargement (27).
